# EUROPEAN PATENT SPECIFICATION

(11) **EP 3 326 533 B1**
(45) Date of publication and mention of the grant of the patent: **11.03.2020**
(21) Application number: 16827910.7
(22) Date of filing: 31.03.2016
(51) Int. Cl.: A61B 6/00, A61B 6/03, G06T 5/00

(54) **TOMOGRAPHIC DEVICE AND TOMOGRAPHIC IMAGE PROCESSING METHOD ACCORDING TO SAME**
TOMOGRAPHISCHE VORRICHTUNG UND VERFAHREN ZUR VERARBEITUNG TOMOGRAPHISCHER BILDER DAMIT
DISPOSITIF TOMOGRAPHIQUE ET PROCÉDÉ DE TRAITEMENT D'IMAGE TOMOGRAPHIQUE ASSOCIÉ

(30) Priority: 17.07.2015 KR 20150101910
(43) Date of publication of application: 30.05.2018
(73) Proprietor: Samsung Electronics Co., Ltd., Suwon-si, Gyeonggi-do 16677 (KR)
(72) Inventor: YOO, Sang-wook, Suwon-si Gyeonggi-do 16527 (KR); LIM, Jae-guyn, Seongnam-si Gyeonggi-do 13612 (KR); KIM, Hyo-geun, Seoul 06354 (KR); YI, Jong-hyon, Yongin-si Gyeonggi-do 16903 (KR); LEE, Gun-woo, Seoul 08208 (KR)
(74) Representative: Gulde & Partner
(86) International application number: PCT/KR2016/003299
(87) International publication number: WO 2017/014406

(56) References cited:
- KR-A- 20140 024 646
- KR-A- 20140 102 515
- KR-A- 20140 130 786
- KR-A- 20150 021 231
- US-A1- 2005 063 611
- US-A1- 2014 050 380
- US-A1- 2014 161 335
- US-A1- 2015 063 716

## Description

### [Technical Field]

The present disclosure relates to tomography apparatuses and methods of processing a tomography image.

### [Background Art]

Medical imaging apparatuses are used to acquire images showing an internal structure of an object. The medical imaging apparatuses are non-invasive examination apparatuses that capture and process images of details of structures, tissue, fluid flow, etc., inside a body and provide the images to a user. A user, e.g., a medical practitioner, may use medical images output from the medical imaging apparatuses to diagnose a patient's condition and diseases. Examples of medical imaging apparatuses are shown in US-2014/0050380 A1 and US-2014/0161335 A1.

A computed tomography (CT) apparatus is a representative example of an apparatus for photographing an object by emitting X-rays toward a patient. In detail, a computed tomography (CT) apparatus may be an example of a tomography apparatus

Among medical image processing apparatuses, a CT apparatus that is a tomography apparatus is capable of providing a cross-sectional image of an object. Furthermore, the CT apparatus may represent an internal structure (e.g., organs such as a kidney, a lung, etc.) of the object without superimposition of adjacent structures, as compared to a general X-ray apparatus. Due to these advantages, a CT apparatus has been widely used for precise diagnosis of diseases. A medical image acquired by a tomography apparatus is hereinafter referred to as a tomography image.

To obtain a tomography image, a tomography apparatus performs a tomography scan of an object to acquire raw data. The acquired raw data is used to reconstruct a tomography image. In this case, the raw data may be projection data obtained by projecting an X-ray onto the object, or may be a projection data set called a sinogram.

Due to movement of a tomography apparatus or an object to be scanned or performance of the tomography apparatus, blurring artifacts may occur during reconstruction of a tomography image. For example, a tomography apparatus itself may undergo shaking during operation, and the shaking may introduce blurring artifacts into an image.

When blurring artifacts occur, an outermost edge of an object may appear unclear in a reconstructed tomography image, and an inner edge of the object in the tomography image may be blurred.

Blurring artifacts in a tomography image reduce the quality of the tomography image and accordingly, degrade the accuracy of analysis of an image and diagnosis of a disease by a user, e.g., a medical practitioner.

Thus, for a tomography scan, it is of paramount importance to minimize blurring artifacts in a tomography image.

### [Disclosure]

### [Technical Problem]

Provided are tomography apparatuses and methods of processing a tomography image which are capable of reducing blurring artifacts that may occur in a reconstructed tomography image.

### [Technical Solution]

A tomography apparatus comprises a controller configured to acquire a point spread function (PSF) map representing a PSF that varies according to a position in a field of view (FOV) created within a gantry, and an image processor configured to acquire a final tomography image by deblurring a tomography image based on the acquired PSF map.

### [Advantageous Effects]

According to embodiments, blurring artifacts in an image may be effectively reduced by deblurring the image based on a PSF map representing PSF that varies according to a position in a FOV created within a gantry of a tomography apparatus.

Furthermore, boosting of a noise component may be prevented by removing a noise component from an image and deblurring the resultant image.

### [Description of Drawings]

These and/or other aspects will become apparent and more readily appreciated from the following description of the embodiments, taken in conjunction with the accompanying drawings in which:
FIG. 1 is a schematic diagram of a general computed tomography (CT) system;
FIG. 2 illustrates a structure of a CT system according to an embodiment;
FIG. 3 illustrates a configuration of a communication unit;
FIGS. 4A and 4B are diagrams for explaining the need for accurate estimation of a point spread function (PSF) and removing noise;
FIG. 5A is a block diagram of a configuration of a tomography apparatus according to an embodiment;
FIG. 5B is a detailed diagram of a field of view (FOV);
FIGS. 6A and 6B are diagrams for explaining a PSF;
FIGS. 7A through 7C are other diagrams for explaining a PSF;
FIG. 8 is a diagram for explaining a process of estimating a PSF;
FIG. 9 is a diagram for explaining a process of generating a PSF map;
FIG. 10 illustrates a configuration of a tomography apparatus according to an embodiment;
FIG. 11 illustrates a process of acquiring a final tomography image from a tomography apparatus, according to an embodiment;
FIG. 12 illustrates a process of acquiring a final tomography image from a tomography apparatus, according to another embodiment;
FIG. 13 illustrates a process of acquiring a final tomography image from a tomography apparatus, according to another embodiment;
FIG. 14 is a flowchart of a method of generating a PSF map according to an embodiment;
FIG. 15 is flowchart of a method of processing a tomography image according to an embodiment; and
FIG. 16 is a diagram for explaining reduction of blurring artifacts in a tomography image, according to an embodiment.

### [Best Mode]

According to an aspect of an embodiment, a tomography apparatus includes: a controller configured to acquire a point spread function (PSF) map representing a PSF that varies according to a position in a field of view (FOV) created within a gantry; and an image processor configured to acquire a final tomography image by deblurring a tomography image based on the acquired PSF map. The image processor is further configured to remove a noise component from the tomography image and to deblur the tomography image from which the noise component is removed, based on the acquired PSF map.

The controller may estimate a plurality of PSFs respectively corresponding to a plurality of different positions, based on a plurality of sample images acquired by performing a tomography scan on a sample object at the plurality of different positions, respectively, and generate the PSF map based on the estimated plurality of PSFs.

The controller may estimate, based on the plurality of sample images, each PSF as a form of a Gaussian function with respect to a position.

The controller may generate the PSF map by applying interpolation to the plurality of PSFs.

The controller may generate the PSF map by applying extrapolation to the plurality of PSFs.

The tomography apparatus may further include a data acquisition device configured to acquire tomography data by performing a tomography scan on an object, and the image processor may reconstruct the tomography image based on the acquired tomography data and acquire the final tomography image by deblurring the tomography image, based on the PSF map.

The image processor may acquire the noise component by generating a difference image between the tomography image and the tomography image from which the noise component is removed and acquire as the final tomography image a sum image generated by adding the acquired noise component to the deblurred tomography image.

The tomography apparatus may further include a storage device configured to store the PSF.

According to an aspect of another embodiment not forming a part of this invention per se, a method of generating a PSF map includes: estimating a plurality of PSFs respectively corresponding to a plurality of different positions, based on a plurality of sample images acquired by performing a tomography scan on a sample object at the plurality of different positions, respectively; and generating, based on the estimated plurality of PSFs, a PSF map representing a PSF that varies according to a position in a FOV created within a gantry.

The estimating of the plurality of PSFs may include estimating, based on the plurality of sample images, each PSF as a form of a Gaussian function with respect to a position.

The generating of the PSF map may include generating the PSF map by applying interpolation to the plurality of PSFs.

The generating of the PSF map may include generating the PSF map by applying extrapolation to the plurality of PSFs.

According to an aspect of another embodiment, a method of processing a tomography image includes: acquiring a PSF map representing a PSF that varies according to a position in a FOV created within a gantry; and acquiring a final tomography image by deblurring the tomography image, based on the acquired PSF map. The acquiring of the final tomography image further comprises removing a noise component from the tomography image and deblurring the tomography image from which the noise component is remoed, based on the acquired PSF map.

The acquiring of the PSF map may include: estimating a plurality of PSFs respectively corresponding to a plurality of different positions, based on a plurality of sample images acquired by performing a tomography scan on a sample object at the plurality of different positions, respectively; and generating the PSF map based on the estimated plurality of PSFs.

The acquiring of the final tomography image may include: reconstructing the tomography image based on tomography data acquired by performing a tomography scan on an object; and acquiring the final tomography image by deblurring the tomography image based on the PSF map.

The acquiring of the final tomography image may include: acquiring a noise component by generating a difference image between the tomography image and the tomography image from which the noise component is removed; and acquiring as the final tomography image a sum image produced by adding the acquired noise component to the deblurred tomography image.

The method may further include storing the PSF.

### [Mode for Invention]

Advantages and features of one or more embodiments of the inventive concept and methods of accomplishing the same may be understood more readily by reference to the following detailed description of the embodiments and the accompanying drawings. In this regard, the present embodiments may have different forms and should not be construed as being limited to the descriptions set forth herein. Rather, these embodiments are provided so that this disclosure will be thorough and complete and will fully convey the concept of the present embodiments to one of ordinary skill in the art, and the inventive concept will only be defined by the appended claims. Like reference numerals refer to like elements throughout the specification.

Hereinafter, the terms used in the specification will be briefly defined, and the embodiments will be described in detail.

All terms including descriptive or technical terms which are used herein should be construed as having meanings that are obvious to one of ordinary skill in the art. However, the terms may have different meanings according to the intention of one of ordinary skill in the art, precedent cases, or the appearance of new technologies. Also, some terms may be arbitrarily selected by the applicant, and in this case, the meaning of the selected terms will be described in detail in the detailed description of the invention. Thus, the terms used herein have to be defined based on the meaning of the terms together with the description throughout the specification.

When a part "includes" or "comprises" an element, unless there is a particular description contrary thereto, the part can further include other elements, not excluding the other elements. Also, the term "unit" in the embodiments of the inventive concept means a software component or hardware component such as a field-programmable gate array (FPGA) or an application-specific integrated circuit (ASIC), and performs a specific function. However, the term "unit" is not limited to software or hardware. The "unit" may be formed so as to be in an addressable storage medium, or may be formed so as to operate one or more processors. Thus, for example, the term "unit" may refer to components such as software components, object-oriented software components, class components, and task components, and may include processes, functions, attributes, procedures, subroutines, segments of program code, drivers, firmware, micro codes, circuits, data, a database, data structures, tables, arrays, or variables. A function provided by the components and "units" may be associated with the smaller number of components and "units", or may be divided into additional components and "units".

Reference will now be made in detail to embodiments, examples of which are illustrated in the accompanying drawings. In this regard, the present embodiments may have different forms and should not be construed as being limited to the descriptions set forth herein. In the following description, well-known functions or constructions are not described in detail so as not to obscure the embodiments with unnecessary detail. Expressions such as "at least one of," when preceding a list of elements, modify the entire list of elements and do not modify the individual elements of the list.

Throughout the specification, an "image" may mean multi-dimensional data formed of discrete image elements, e.g., pixels in a two-dimensional (2D) image and voxels in a three-dimensional (3D) image. For example, the image may include a medical image of an object which is captured by a computed tomography (CT) imaging apparatus.

Throughout the specification, a "CT image" may mean an image generated by synthesizing a plurality of X-ray images that are obtained by photographing an object while a CT imaging apparatus rotates around at least one axis with respect to the object.

Throughout the specification, an "object" may be a human, an animal, or a portion of a human or animal. For example, the object may be an organ (e.g., the liver, heart, womb, brain, breast, or abdomen), a blood vessel, or a combination thereof. The object may be a phantom. The phantom means a material having a density, an effective atomic number, and a volume that are approximately the same as those of an organism. For example, the phantom may be a spherical phantom having properties similar to the physical body.

Throughout the specification, a "user" may be, but is not limited to, a medical expert including a medical doctor, a nurse, a medical laboratory technologist, a medial image expert, or a technician who repairs a medical apparatus.

Since a CT system is capable of providing a cross-sectional image of an object, the CT system may distinctively express an inner structure, e.g., an organ such as a kidney or a lung, of the object, compared to a general X-ray imaging apparatus.

In detail, a tomography system may include all tomography apparatuses such as a computed tomography (CT) apparatus, an optical coherence tomography (OCT), or a positron emission tomography (PET)-CT apparatus.

In the following description, a CT system is exemplified as the tomography system.

The CT system may obtain a plurality of pieces of image data with a thickness not more than 2 mm several hundred times per second and then may process the plurality of pieces of image data, so that the CT system may provide a relatively accurate cross-sectional image of the object. According to the related art, only a horizontal cross-sectional image of the object can be obtained, but this issue has been overcome due to various image reconstruction methods. Examples of 3D image reconstruction methods are as below:
Shade surface display (SSD) - an initial 3D imaging method of displaying only voxels having a predetermined Hounsfield Units (HU) value.

Maximum intensity projection (MIP)/minimum intensity projection (MinIP) - a 3D imaging method of displaying only voxels having the greatest or smallest HU value from among voxels that construct an image.

Volume rendering (VR) - an imaging method capable of adjusting a color and transmittance of voxels that constitute an image, according to areas of interest.

Virtual endoscopy - a method that allows endoscopy observation in a 3D image that is reconstructed by using the VR method or the SSD method.

Multi-planar reformation (MPR) - a method of reconstructing an image into a different cross-sectional image. A user may reconstruct an image in any desired direction.

Editing - a method of editing adjacent voxels so as to allow a user to easily observe an area of interest in volume rendering.

Voxel of interest (VOI) - a method of displaying only a selected area in volume rendering.

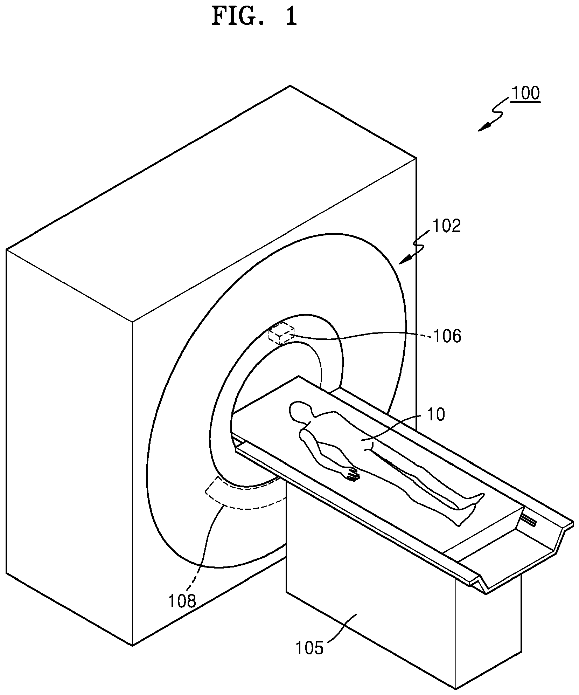

A CT system 100 according to an embodiment of the present invention will now be described with reference to FIG. 1. The CT system 100 may include various types of devices.

FIG. 1 schematically illustrates the CT system 100.

Referring to FIG. 1, the CT system 100 may include a gantry 102, a table 105, an X-ray generating unit 106, and an X-ray detecting unit 108.

The gantry 102 may include the X-ray generator 106 and the X-ray detector 108.

An object 10 may be positioned on the table 105.

The table 105 may move in a predetermined direction (e.g., at least one of up, down, right, and left directions) during a CT imaging procedure. Also, the table 105 may tilt or rotate by a predetermined angle in a predetermined direction.

The gantry 102 may also tilt by a predetermined angle in a predetermined direction.

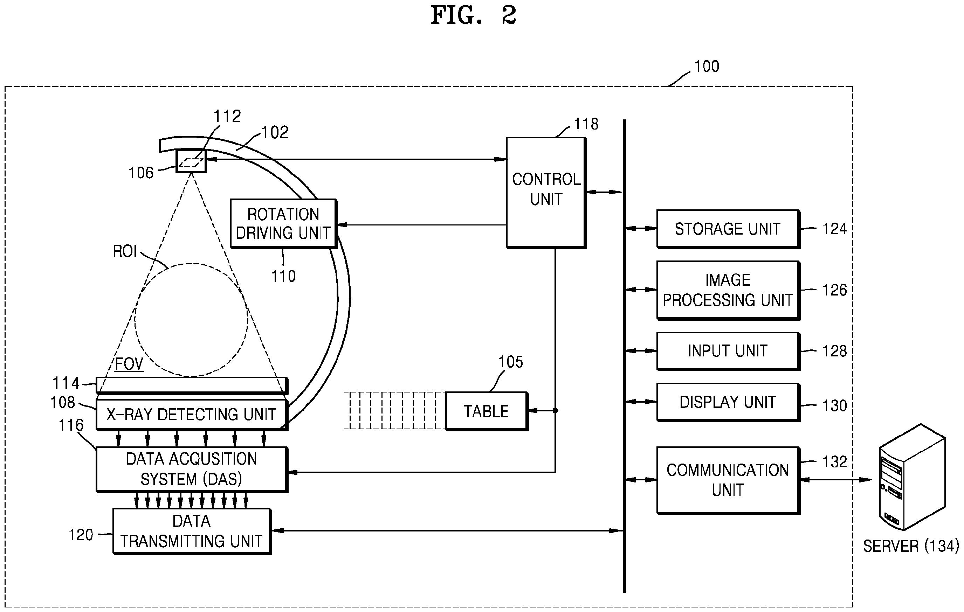

FIG. 2 is a block diagram illustrating a structure of the CT system 100.

The CT system 100 may include the gantry 102, the table 105, a control unit 118, a storage unit 124, an image processing unit 126, an input unit 128, a display unit 130, and a communication unit 132.

As described above, the object 10 may be positioned on the table 105. In the present embodiment, the table 105 may move in a predetermined direction (e.g., at least one of up, down, right, and left directions), and movement of the table 105 may be controlled by the control unit 118.

The gantry 102 may include a rotating frame 104, the X-ray generating unit 106, the X-ray detecting unit 108, a rotation driving unit 110, a data acquisition system (DAS) 116, and a data transmitting unit 120.

The gantry 102 may include the rotating frame 104 having a loop shape capable of rotating with respect to a predetermined rotation axis RA. Also, the rotating frame 104 may have a disc shape.

The rotating frame 104 may include the X-ray generator 106 and the X-ray detector 108 that are arranged to face each other so as to have predetermined fields of view FOV. The rotating frame 104 may also include an anti-scatter grid 114. The anti-scatter grid 114 may be positioned between the X-ray generating unit 106 and the X-ray detecting unit 108.

In a medical imaging system, X-ray radiation that reaches a detector (or a photosensitive film) includes not only attenuated primary radiation that forms a valuable image but also scattered radiation that deteriorates the quality of an image. In order to transmit most of the primary radiation and to attenuate the scattered radiation, the anti-scatter grid 114 may be positioned between a patient and the detector (or the photosensitive film).

For example, the anti-scatter grid 114 may be formed by alternately stacking lead foil strips and an interspace material such as a solid polymer material, solid polymer, or a fiber composite material. However, formation of the anti-scatter grid 114 is not limited thereto.

The rotating frame 104 may receive a driving signal from the rotation driving unit 110 and may rotate the X-ray generator 106 and the X-ray detector 108 at a predetermined rotation speed. The rotating frame 104 may receive the driving signal and power from the rotation driving unit 110 while the rotating frame 104 contacts the rotation driving unit 110 via a slip ring (not shown). Also, the rotating frame 104 may receive the driving signal and power from the rotation driving unit 110 via wireless communication.

The X-ray generator 106 may receive a voltage and current from a power distribution unit (PDU) (not shown) via a slip ring (not shown) and then a high voltage generating unit (not shown), and may generate and emit an X-ray. When the high voltage generating unit applies predetermined voltage (hereinafter, referred to as a tube voltage) to the X-ray generator 106, the X-ray generator 106 may generate X-rays having a plurality of energy spectra that correspond to the tube voltage.

The X-ray generated by the X-ray generator 106 may be emitted in a predetermined form due to a collimator 112.

The X-ray detector 108 may be positioned to face the X-ray generator 106. The X-ray detector 108 may be positioned to face the X-ray generator 106. Each of the plurality of X-ray detecting devices may establish one channel but one or more embodiments of the present invention are not limited thereto.

The X-ray detector 108 may detect the X-ray that is generated by the X-ray generator 106 and that is transmitted through the object 10, and may generate an electrical signal corresponding to intensity of the detected X-ray.

The X-ray detector 108 may include an indirect-type X-ray detector for detecting radiation after converting the radiation into light, and a direct-type X-ray detector for detecting radiation after directly converting the radiation into electric charges. The indirect-type X-ray detector may use a scintillator. Also, the direct-type X-ray detector may use a photon counting detector. The DAS 116 may be connected to the X-ray detector 108. Electrical signals generated by the X-ray detector 108 may be acquired by the DAS 116. Electrical signals generated by the X-ray detector 108 may be acquired by wire or wirelessly by the DAS 116. Also, the electrical signals generated by the X-ray detector 108 may be provided to an analog-to-digital converter (not shown) via an amplifier (not shown).

According to a slice thickness or the number of slices, only some of a plurality of pieces of data collected by the X-ray detector 108 may be provided to the image processing unit 126 via the data transmitting unit 120, or the image processing unit 126 may select only some of the plurality of pieces of data.

Such a digital signal may be provided to the image processing unit 126 via the data transmitting unit 120. The digital signal may be provided to the image processing unit 126 by wire or wirelessly.

The control unit 118 may control an operation of each of the elements in the CT system 100. For example, the control unit 118 may control operations of the table 105, the rotation driving unit 110, the collimator 112, the DAS 116, the storage unit 124, the image processing unit 126, the input unit 128, the display unit 130, the communication unit 132, or the like.

The image processing unit 126 may receive data acquired by the DAS 116 (e.g., raw data that is data before processing), via the data transmitting unit 120, and may perform pre-processing.

The pre-processing may include, for example, a process of correcting a sensitivity irregularity between channels and a process of correcting signal loss due to a rapid decrease in signal strength or due to the presence of an X-ray absorbing material such as metal.

Data output from the image processing unit 126 may be referred to as raw data or projection data. The projection data may be stored in the storage unit 124 with imaging conditions (e.g., the tube voltage, an imaging angle, etc.) during the acquisition of data.

The projection data may be a group of data values that correspond to the intensity of the X-ray that has passed through the object 10. For convenience of description, a group of a plurality of pieces of projection data that are simultaneously obtained from all channels at the same imaging angle is referred to as a projection data set.

The storage unit 124 may include at least one storage medium from among a flash memory-type storage medium, a hard disk-type storage medium, a multimedia card micro-type storage medium, card-type memories (e.g., an SD card, an XD memory, and the like), random access memory (RAM), static random access memory (SRAM), read-only memory (ROM), electrically erasable programmable ROM (EEPROM), programmable ROM (PROM), magnetic memory, a magnetic disc, and an optical disc.

The image processing unit 126 may reconstruct a cross-sectional image of the object 10 by using the acquired projection data set. The cross-sectional image may be a 3D image. In other words, the image processing unit 126 may reconstruct a 3D image of the object 10 by using a cone beam reconstruction method or the like, based on the acquired projection data set.

The input unit 128 may receive an external input with respect to an X-ray tomography imaging condition, an image processing condition, or the like. For example, the X-ray tomography imaging condition may include tube voltages, an energy value setting with respect to a plurality of X-rays, a selection of an imaging protocol, a selection of an image reconstruction method, a setting of a FOV area, the number of slices, a slice thickness, a parameter setting with respect to image post-processing, or the like. Also, the image processing condition may include a resolution of an image, an attenuation coefficient setting for the image, setting for an image combining ratio, or the like.

The input unit 128 may include a device for receiving a predetermined input from an external source. For example, the input unit 128 may include a microphone, a keyboard, a mouse, a joystick, a touch pad, a touch pen, a voice recognition device, a gesture recognition device, or the like.

The display unit 130 may display an X-ray image reconstructed by the image processing unit 126.

Exchanges of data, power, or the like between the aforementioned elements may be performed by using at least one of wired communication, wireless communication, and optical communication.

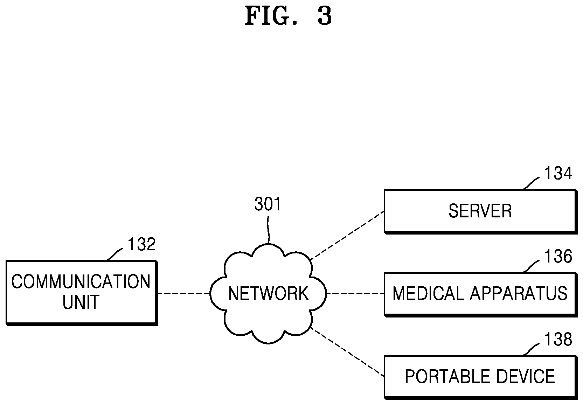

The communication unit 132 may perform communication with an external device, an external medical apparatus, etc. via a server 134 or the like. The communication will now be described with reference to FIG. 3.

FIG. 3 is a block diagram illustrating the communication performed by the communication unit 132.

The communication unit 132 may be wiredly or wirelessly connected to a network 301 and therefore may perform communication with the server 134, a medical apparatus 136, or a portable device 138. The communication unit 132 may exchange data with a hospital server or other medical apparatuses in a hospital connected via a picture archiving and communication system (PACS).

Also, the communication unit 132 may perform data communication with the portable device 138 or the like, according to the digital imaging and communications in medicine (DICOM) standard.

The communication unit 132 may transmit and receive data related to diagnosing the object 10, via the network 301. Also, the communication unit 132 may transmit and receive a medical image obtained from the medical apparatus 136 such as a magnetic resonance imaging (MRI) apparatus, an X-ray apparatus, or the like.

Furthermore, the communication unit 132 may receive a diagnosis history or a medical treatment schedule about a patient from the server 134 and may use the diagnosis history or the medical treatment schedule to diagnose the patient. Also, the communication unit 132 may perform data communication not only with the server 134 or the medical apparatus 136 in a hospital but also with the portable device 138 of a user or patient.

Also, the communication unit 132 may transmit information about a device error, information about a quality control status, or the like to a system manager or a service manager via the network 301, and may receive a feedback regarding the information from the system manager or service manager.

All imaging apparatuses have specific spatial resolutions. A spatial resolution refers to the precision of an image captured using an imaging apparatus driven to photograph an object in a space. Due to characteristics of an imaging apparatus, an image acquired by the imaging apparatus may not perfectly represent a state of an object at a point of time when the image is captured. In other words, due to shaking of the imaging apparatus that occurs while being driven, the captured image itself may also be blurred. Thus, a spatial resolution is determined based on the degree of blurring induced in a captured image. For example, an imaging apparatus having a high spatial resolution suffers from a smaller degree of blurring in an image than an imaging apparatus having a low spatial resolution.

A tomography apparatus has a specific spatial resolution. A limitation with respect to the spatial resolution of the tomography apparatus leads to blurring artifacts occurring in a tomography image. These blurring artifacts in a tomography image reduce the quality of the tomography image and accordingly, degrade the accuracy of analysis of an image and diagnosis of a disease by a user, e.g., a medical practitioner. For example, if blurring artifacts occur in a portion of an image representing a calcium region, a blood vessel may appear to be blocked even if it is actually not. Thus, the accuracy of diagnosis of a vascular disease may be lowered.

Blurring artifacts may be reduced by estimating a point spread function (PSF) and deblurring an image based on the estimated PSF. In this case, a PSF varies from one tomography apparatus to another. In detail, a PSF may vary according to specifications and/or performance of a tomography apparatus. The PSF may have a complex shape and vary according to a position in a field of view (FOV) created within a gantry and a tube current (measured in miliamperes) for generating X-rays. In this case, reduction of blurring artifacts may be referred to as 'de-blurring' or 'de-blooming'. Hereinafter, for convenience of explanation, correction of blurring artifacts is commonly referred to as de-blurring.

When an image is deblurred based on a PSF, inaccurate estimation of the PSF may result in generation of an image from which blurring has not been completely removed. Furthermore, the inaccurate estimation of the PSF may cause artifacts such as undershooting or overshooting in a deblurred image.

In general, one PSF is estimated and used to deblur an image. In other words, a captured image is deblurred by applying one specific PSF to a particular imaging apparatus. When an image is deblurred based on the same PSF at all positions (in a FOV, inaccurate PSFs may be applied at some of the locations, which may result in generation of an image from which blurring has not been completely removed or introduce artifacts such as undershooting or overshooting into an image.

However, a PSF may vary according to a position in a FOV of a particular tomography apparatus. Thus, according to an embodiment, a PSF is predicted which varies according to a position in a FOV created within a gantry of a tomography apparatus and is used for deblurring. In other words, a PSF that varies according to a position in a FOV is predicted to achieve an accurate PSF estimation.

Furthermore, a reconstructed image contains a noise component as well as a component representing an object to be imaged. When a tomography apparatus performs deblurring on an image without separately processing a noise component from the image, the noise component as well as the component representing the object may be deblurred, and thus the noise component may actually be boosted. Thus, a noise component needs to be processed separately from a component representing an object by removing the noise component from an image.

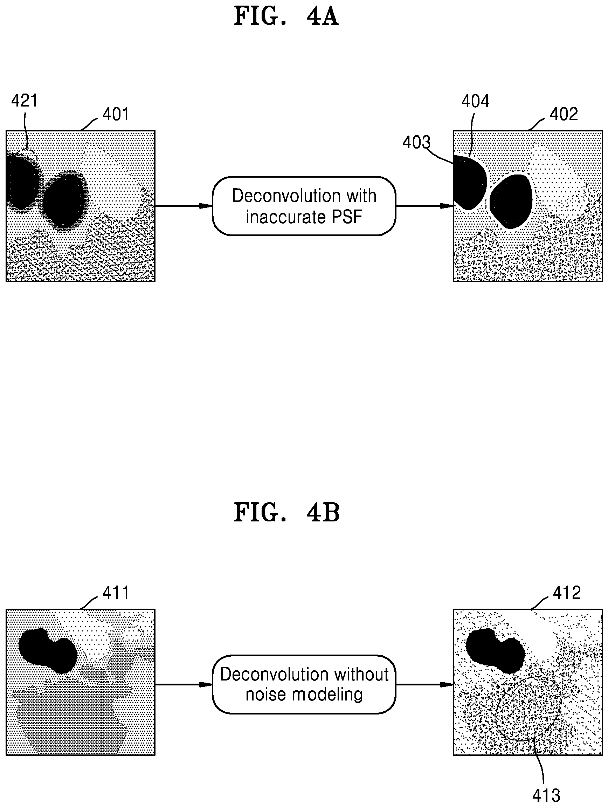

FIGS. 4A and 4B are diagrams for explaining the need for accurate PSF estimation and removing noise.

Referring to FIGS. 4A and 4B, initial images 401 and 411 respectively shown in FIGS. 4A and 4B are images obtained before being deblurred. In this case, due to blurring artifacts in the initial images 401 and 411, portions in the initial images 401 and 411 representing contours of an object may appear blurry. Referring to a region 421 shown in FIG. 4A, a contour of an object is not represented by one distinct gray level but by a plurality of gray levels having similar values. Thus, the contour of the object is not clearly depicted in the initial image 401.

A deblurred image 402 shown in FIG. 4A is obtained based on an inaccurate PSF. In this case, a portion in the deblurred image 402 representing a contour of the object 403 appears clearer than that in the initial image 401. However, artifacts having a form of a white band occur in a portion 404 representing an edge of the object 403.

A deblurred image 412 shown in FIG. 4B is obtained by deblurring the initial image 411 without removing a noise component from the initial image 411. In this case, blurring artifacts may be reduced, but at the same time the noise component may be boosted. Accordingly, a component representing an object as well as a noise component 413 may be clearly shown in the deblurred image 412.

Thus, to effectively reduce blurring artifacts during a tomography scan of an object, it is necessary to deblur an image based on an accurately estimated PSF and separate a component representing an object from a noise component for processing.

According to embodiments, blurring artifacts in an image may be effectively reduced by deblurring the image based on a PSF map representing PSF that varies according to a position in a FOV created within a gantry of a tomography apparatus. Furthermore, boosting of a noise component may be prevented by removing a noise component from an image and deblurring the resultant image.

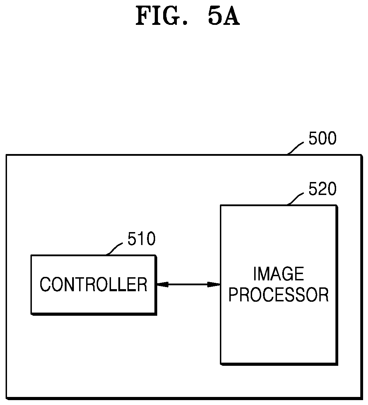

FIG. 5A is a block diagram of a configuration of a tomography apparatus 500 according to an embodiment

Referring to FIG. 5A, the tomography apparatus 500 according to the present embodiment includes a controller 510 and an image processor 520. The tomography apparatus 500 may be any type of electronic devices that are capable of acquiring, reconstructing, and/or displaying a tomography image by performing tomography scanning.

The tomography apparatus 500 may be included in the CT system 100 described with reference to FIGS. 1 and 2. In this case, the controller 510 and the image processor 520 may correspond to the control unit 118 and the image processing unit 126 shown in FIG. 2. Furthermore, the tomography apparatus 500 may be included in the medical apparatus 136 or the portable device 138 described with reference to FIG. 3 and be connected to the CT system 100 to be operated.

According to an embodiment, the controller 510 may obtain a PSF map representing a PSF that varies according to a position in a FOV.

The PSF map may be generated based on a plurality of PSFs respectively corresponding to a plurality of positions in the FOV. In this case, the plurality of PSFs may be acquired from outside the tomography apparatus 500. Alternatively, the tomography apparatus may acquire a plurality of sample images by performing a tomography scan on a sample object at a plurality of different positions, respectively, and estimate a plurality of PSFs respectively corresponding to the plurality of different positions.

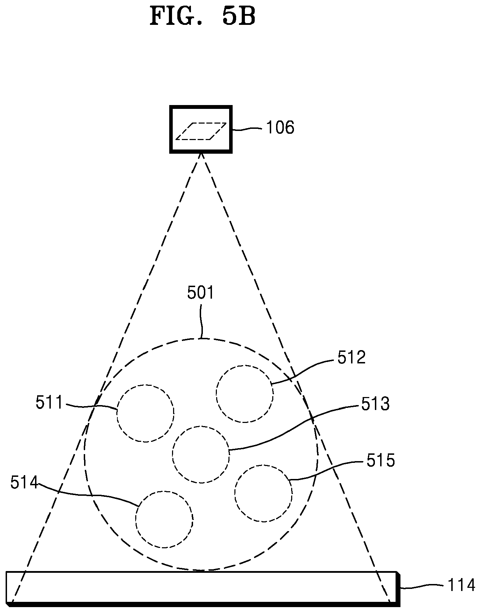

FIG. 5B is a detailed diagram of the FOV shown in FIG. 2. In FIG. 5B, the same elements as those shown in FIG. 2 are denoted by the same reference numbers.

Referring to FIG. 5B, according to an embodiment, the tomography apparatus 500 performs tomography scanning on a sample object that is placed at each of a plurality of different positions 511 through 515. During the tomography scan, the tomography apparatus 500 may acquire a plurality of sample images respectively corresponding to the plurality of different positions 511 through 515. Although the plurality of different positions 511 through 515 are set at the positions shown in FIG. 5B, they may be set at positions different from those shown in FIG. 5B. Furthermore, while FIG. 5 shows that the plurality of different positions 511 through 515 do not overlap one another, all or some of the plurality of different positions 511 through 515 may overlap one another.

The controller 510 may estimate a plurality of PSFs respectively corresponding to a plurality of positions based on a plurality of sample images.

For example, the controller 510 estimate a first PSF based on a first sample image acquired by placing a sample object at the position 511 and performing a tomography scan thereon and a second PSF based on a second sample image acquired by placing the sample object at the position 512 and performing a tomography scan thereon. In the same manner, the controller 510 may estimate a plurality of PSFs respectively corresponding to the plurality of different positions 511 through 515.

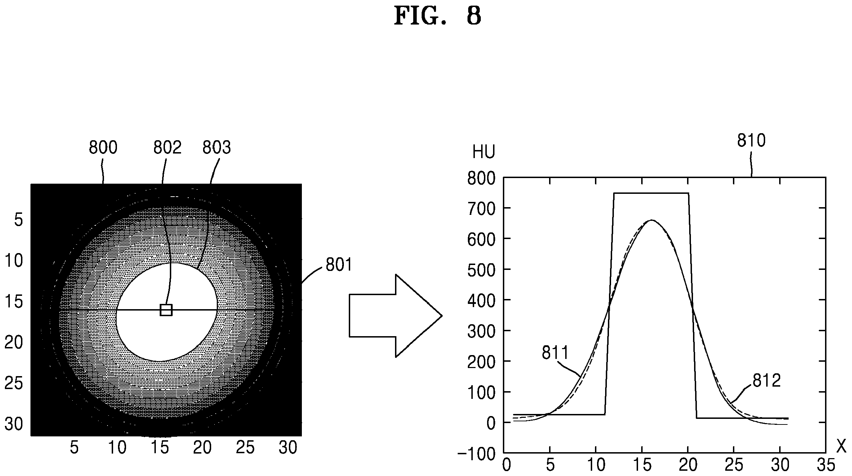

Furthermore, the controller 510 may estimate a PSF as a form of a Gaussian function with respect to a position. However, a method of estimating a PSF is not limited to estimation as a form of a Gaussian function and will be described in more detail below with reference to FIG. 8.

According to an embodiment, the controller 510 may generate a PSF map corresponding to a position in a FOV formed within a gantry, based on the estimated plurality of PSFs. In this case, the PSF map may vary according to a position in a FOV. Furthermore, the PSF map may include a single PSF corresponding to a center of a FOV or a plurality of PSFs respectively corresponding to a plurality of pixels in an image. The number of PSFs in the PSF map may vary according to settings. As the number of PSFs in the PSF map increases, an image may be deblurred based on more accurate PSFs with respect to a position.

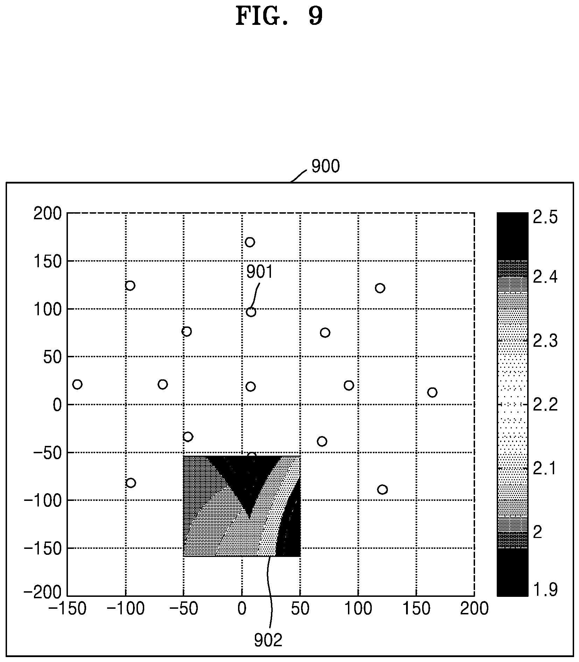

According to another embodiment, the controller 510 may generate a PSF map by applying interpolation or extrapolation to a plurality of PSFs, as described in more detail below with reference to FIG. 9.

According to an embodiment, the image processor 520 may perform deblurring on a tomography image based on the generated PSF map to acquire a final tomography image. In detail, the image processor 520 may deblur a tomography image by performing deconvolution on the tomography image. Since deconvolution of an image is widely known to those of ordinary skill in the art, a detailed description thereof will be omitted below.

According to another embodiment, the image processor 520 may remove a noise component from a tomography image and deblur the resultant image based on a PSF map. Removing of the noise component may be performed before or after deblurring the resultant image.

Furthermore, the image processor 520 may generate a difference image between a tomography image and an image from which a noise component is removed, in order to acquire an image that appears more natural to a user. The image processor 520 may generate a sum image by adding the acquired noise component to a deblurred image and acquire the sum image as a final tomography image.

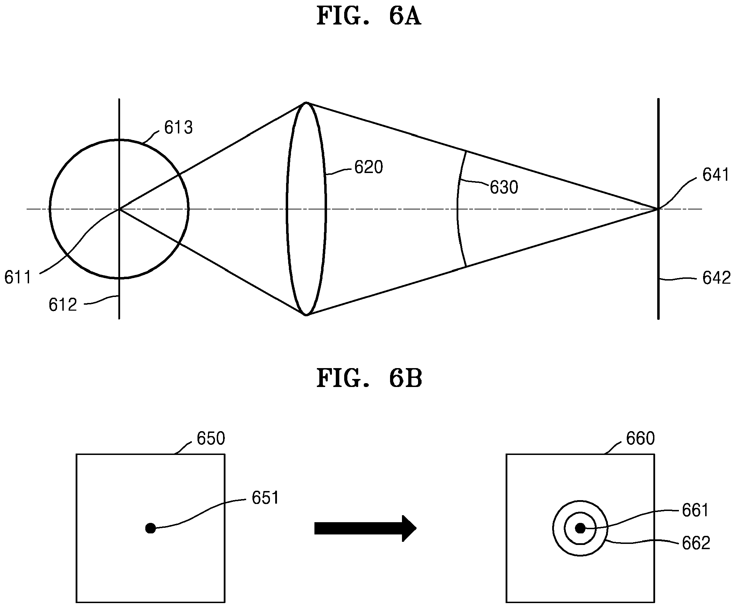

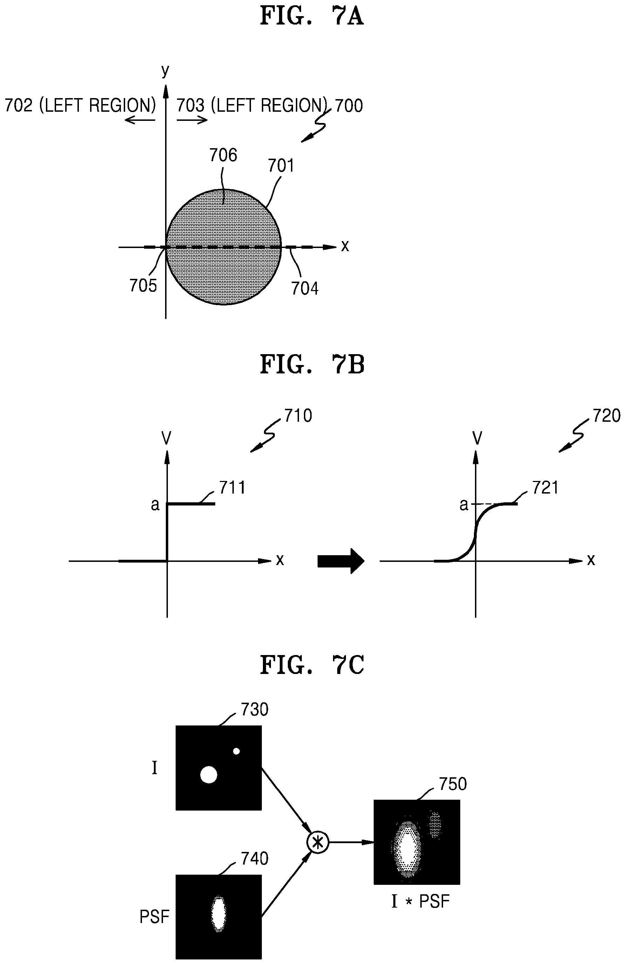

A PSF will now be described in more detail with reference to FIGS. 6A and 6B and 7A through 7C.

FIGS. 6A and 6B are diagrams for explaining a PSF.

A PSF is a function that describes a spatial response of an imaging system with respect to a point. In other words, a PSF is a spatial impulse response of an imaging system. In this case, the PSF may closely approximate a Gaussian function.

Referring to FIG. 6A, a wave for imaging a point 611 of an object in an object plane 612 (indicated by a straight line for convenience) may be transmitted. For example, the wave may be emitted from the point 611 in a spherical form 613. When an imaging system 620 is an ultrasound system, the wave may be an ultrasound wave. When the imaging system 620 is an optical apparatus such as a microscope, the wave may be light. When the imaging system 620 is a CT or X-ray apparatus, the wave may be an X-ray. The imaging system 620 may obtain a portion 630 of the wave in the form of the sphere 613.

Furthermore, in the imaging system 620, the point 611 in the object plane 612 may be imaged onto a point 641 in an image plane 642.

Referring to FIG. 6B, a point 651 in an object plane 650 may be imaged onto a point 661 in an image plane 660. Furthermore, artifacts 662 may occur in the image plane 660. The artifacts 662 may be blurring artifacts and be circular.

FIGS. 7A through 7C are other diagrams for explaining a PSF.

FIG. 7A shows an image 700 representing an object 706 in a plane. Rectangular coordinate axes may be set in the image 700. For example, an X-axis may be set to pass through the object 706, and a y-axis may be set to be tangent to the object 706.

Referring to FIG. 7B, an x-axis and a v-axis denote a position and a pixel value of each point, respectively. A graph 710 shown in FIG. 7B represents pixel values in a one-dimensional (1D) straight line 704 included in the image 700 when blurring artifacts do not occur in the image 700.

In the graph 710, a coordinate plane is divided into a left region 702, in which an x-coordinate is positive, and a right region 703, in which an x-coordinate is negative. Furthermore, an original point corresponds to a point 705 included in a surface 701. In the example of FIG. 7B, as shown in the graph 710, a pixel value is '0' if an x-coordinate is negative and is 'a' if an x-coordinate is positive. Thus, as apparent from FIG. 7B, the image 700 has a clear contour when an x-coordinate is 0.

Furthermore, a graph 720 shown in FIG. 7B is a transformation of the graph 710by a predetermined PSF. The predetermined PSF may be a PSF present in the tomography apparatus 500.

As apparent from the graph 720, a pixel value may gradually change near an x-coordinate of 0 due to a PSF. Thus, the tomography apparatus 500 may have difficulty in acquiring a contour from the 1 D image.

FIG. 7C shows 2D images.

Referring to FIG. 7C, an image 730 shows an object in a 2D space and has no blurring artifacts.

An image 740 shown in FIG. 7C represents a PSF of the tomography apparatus 500. The image 740 has blurring artifacts and has a vertically elongated shape.

When the tomography apparatus 500 photographs an object, the PSF of the tomography apparatus may be applied to acquire an image 750 having blurring artifacts. In detail, the tomography apparatus 500 may convolve the image 730 with the image 740 to acquire the image 750 containing blurring artifacts.

When a PSF is applied to the image 730 including two circles as an object, the image 750 including two blurred elongated circles may be obtained. The tomography apparatus 500 may acquire the PSF of the tomography apparatus 500 by using the image 730 having no blurring artifacts and the image 750 affected by the PSF.

The tomography apparatus may perform deblurring on an image based on the acquired PSF.

In detail, the tomography apparatus 500 may mathematically calculate an inverse PSF based on the acquired PSF. The tomography apparatus 500 may estimate the image 730 having no blurring artifacts by convolving the inverse PSF with the image 750 having blurring artifacts.

Alternatively, the tomography apparatus 500 may estimate the image 730 having no blurring artifacts by deconvolving the image 750 having blurring artifacts with the PSF.

Various methods are used to estimate a PSF. For example, the tomography apparatus 500 may store information about an original shape of a contour of an object. However, embodiments are not limited thereto.

The tomography apparatus 500 may receive information about an original shape of a contour of an object from outside. The information about the original shape of the contour may be information indicating that a pixel value changes rapidly when an x-coordinate is 0. Furthermore, the tomography apparatus 500 may acquire the entire image of the object. The tomography apparatus 500 may acquire a first region having little motion from the entire image. The tomography apparatus 500 may acquire information about a contour of the object from the first region. For example, as shown in the graph 720, the information about the contour of the photographed object may be information indicating that a pixel value changes gradually when an x-coordinate is 0. The tomography apparatus 500 may estimate a PSF based on that information about the contour of the photographed object and the information about the original shape of the contour. In detail, the tomography apparatus 500 may estimate a PSF by convolving an inverse of the image 730 that is the information about the original shape of the contour with the image 750 that is the information about the contour of the photographed object.

A method of estimating a PSF will now be described in detail with reference to FIG. 8.

Referring to FIG. 8, an image 800 is a CT cross-sectional image obtained by performing a tomography scan on a ball-shaped phantom 803 that is a sample object. In the image 800, the abscissa and ordinate respectively denote a position of each of pixels that constitute the image 800. For example, the image 800 may correspond to a 2D plane that is a plane of a FOV 501 described with reference to FIG. 5B. In this case, blurring artifacts may be introduced into the image 800 due to a PSF of the tomography apparatus 500.

According to an embodiment, the controller 510 may measure a distribution 812 of intensities with respect to a position 802 that is a position of a specific pixel on a reference line 801 set within the image 800. In detail, the intensities may be expressed in Hounsfield Units (HU). In this case, the distribution of intensities (HU values) may be represented as a Gaussian function.

The controller 510 may estimate a PSF 811 at the position 802 based on the distribution 812 of intensities. In this case, the estimated PSF 811 may also be represented as a Gaussian function.

According to an embodiment, the controller 510 may acquire a plurality of sample images by performing a tomography scan on a sample object at a plurality of different positions within a gantry of the tomography apparatus 500, respectively. Images captured at different positions may have different types of blurring artifacts. Thus, the images may exhibit different distributions of HU values, and a PSF estimated based on a distribution of HU values may also vary according to a position.

The controller 510 may generate a PSF map corresponding to a position in a FOV created in the gantry, based on a plurality of PSFs respectively corresponding to a plurality of positions.

A method of generating a PSF map will now be described in detail with reference to FIG. 9.

FIG. 9 is a diagram for explaining a process of generating a PSF map based on a plurality of PSFs.

Referring to FIG. 9, a plurality of points 901 shown in a graph 900 represent positions corresponding to a plurality of PSFs estimated by the controller 510. The abscissa and the left ordinate of the graph 900 represent a position in a FOV that is a position in the gantry of the tomography apparatus 500. In detail, a 2D space shown in FIG. 9 corresponds to a 2D space including a FOV, and a position corresponding to a point (0, 0) represents a center of the FOV in the gantry.

Referring to FIG. 9, it can be seen that the plurality of PSFs may be estimated at different positions in the gantry. As the number of estimated PSFs increases, the controller 510 may produce a more accurate PSF map based on the estimated PSFs.

The positions where PSFs are estimated may vary according to settings. Furthermore, the positions may be selected to cover as many regions in the gantry as possible.

Furthermore, an image 902 shown in the graph 900 represents a PSF map corresponding to a FOV. Furthermore, the right ordinate of the graph 900 represents a numerical value of intensity information of the image 902. In this case, a position of the image 902 may represent a position in the FOV created in the gantry.

The controller 510 may generate a PSF map corresponding to a position in a FOV, based on a plurality of sample PSFs. In this case, the PSF map may be considered as a set of PSFs corresponding to positions in the FOV.

For example, the controller 510 may acquire PSFs respectively corresponding to all pixel positions in the image 902, based on a plurality of PSFs. In this case, a PSF map may be a set of the PSFs respectively corresponding to all of the pixel positions.

As another example, the controller 510 may acquire a PSF corresponding to a center of a FOV, based on a plurality of PSFs. The PSF corresponding to the center of the FOV may be a representative PSF that is used to deblur the image 902. In this case, a PSF map may be the PSF corresponding to the center of the FOV. However, as described above, to effectively reduce blurring artifacts, accurate estimation of a PSF that varies depending on a position is required. Thus, as the number of PSFs in a PSF map increases, blurring artifacts in an image may be effectively reduced.

According to an embodiment, the controller 510 may generate a PSF map by applying interpolation or extrapolation to a plurality of PSFs near positions in a FOV, but embodiments are not limited thereto. For example, a single PSF map may be generated by interpolating between a plurality of PSFs. Since the use of interpolation or extrapolation is widely known to those of ordinary skill in the art, a detailed description thereof will be omitted below.

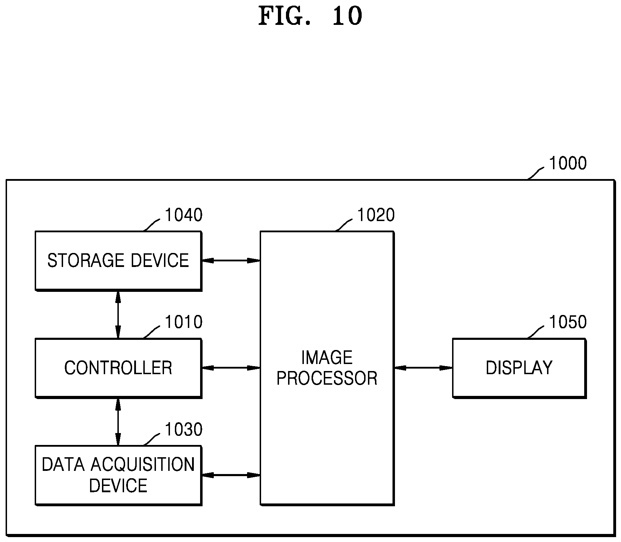

FIG. 10 is a block diagram of a tomography apparatus 1000 according to another embodiment.

Referring to FIG. 10, the tomography apparatus 1000 according to the present embodiment may include a controller 1010, an image processor 1020, a data acquisition device 1030, a storage device 1040, and a display 1050. In this case, since the controller 1010 and the image processor 1020 respectively correspond to the controller 510 and the image processor 520, the same descriptions as already provided with respect to FIG. 5A will be omitted below.

According to an embodiment, the data acquisition device 1030 may acquire tomography data by performing a tomography scan on the object. In detail, the tomography data may be raw data. In this case, the raw data may be projection data obtained by projecting an X-ray onto the object or a projection data set called a sinogram. Furthermore, the raw data may be an image produced by performing filtered backprojection (FBP) on projection data or a sinogram.

Furthermore, according to an embodiment, the data acquisition device 1030 may include a gantry that performs a tomography scan of the object included in a FOV.

According to an embodiment, the image processor 1020 may reconstruct a tomography image based on tomography data acquired by the data acquisition device 1010. In this case, the image processor 1020 may use a full reconstruction method whereby a tomography image is reconstructed from tomography data acquired as the X-ray generating unit 106 rotates by 360°. Furthermore, the image processor 1020 may use a half reconstruction method whereby a tomography image is reconstructed from tomography data acquired as the X-ray generating unit 106 rotates by an angle that is greater than or equal to 180° and less than 360°. However, a method of reconstructing a tomography image is not limited to the half and full reconstruction methods.

The image processor 1020 may also remove a noise component from a tomography image and deblur the resultant tomography image based on a generated PSF map.

In this case, the image processor 1020 may adjust a size of the PSF map according to a FOV in a tomography image to be reconstructed and perform deblurring using the tomography image and the PSF map having the same size. In detail, if a FOV in a tomography image to be reconstructed has a size of a 512 ^{∗} 512 matrix, the image processor 1020 may adjust a size of a PSF map to a size of the 512 ^{∗} 512 matrix with respect to a center of the FOV and perform_deblurring on the tomography image by using the PSF map whose size has been adjusted.

In this case, since a noise filtering method that is generally known to those of ordinary skill in the art may be used to removing the noise component, a detailed description thereof will be omitted below.

The image processor 1020 may perform deblurring on an image from which a noise component is removed, based on the generated PSF map.

As described above, a blurry image may be acquired by performing convolution of an image with a PSF. Thus, deblurring a blurry image may be represented as deconvolution of the blurry image and a PSF.

According to an embodiment, the image processor 1020 may process a noise component separately from a portion representing an object by removing the noise component from an image. Due to this process configuration, the tomography apparatus 1000 may prevent boosting of the noise component in the image.

According to an embodiment, the image processor 1020 may acquire a noise component by generating a difference image between a tomography image and an image from which the noise component is removed. The image processor 1020 may also generate a sum image by adding the acquired noise component to a deblurred image and acquire the sum image as a final tomography image. Due to this configuration, the tomography apparatus 1000 may acquire a tomography image that appears more natural to a user.

When an image from which noise is removed is deblurred, a high quality tomography image may be acquired since a noise component has been greatly removed therefrom. However, a final deblurred image acquired after removing a noise component therefrom may seem unnatural to a user diagnosing an object based on the acquired final image. To solve this problem, the tomography apparatus 1000 may acquire an image that appears more natural to the user by generating a sum image that is generated by adding a noise component to a deblurring image from which the noise component is removed.

According to an embodiment, the storage device 1040 may store a plurality of PSFs estimated by the controller 1010. In detail, the tomography apparatus 1000 may prestore a plurality of PSFs in the storage device 1040. In this case, the controller 1010 may generate a PSF map corresponding to a position in a FOV based on the plurality of PSFs stored in the storage device 1040. Storing an estimated plurality of PSFs in the storage device 1040 may be performed during an initial manufacturing process for the tomography apparatus 1000 or during a calibration process after replacing components within the tomography apparatus 1000. However, embodiments are not limited thereto.

According to an embodiment, the storage device 1040 may store a plurality of PSFs in the form of tables, but is not limited thereto.

According to an embodiment, the display 1050 may output a screen including a final tomography image acquired by the image processor 1020. Furthermore, the display 1050 may display a user interface screen necessary for performing a tomography scan.

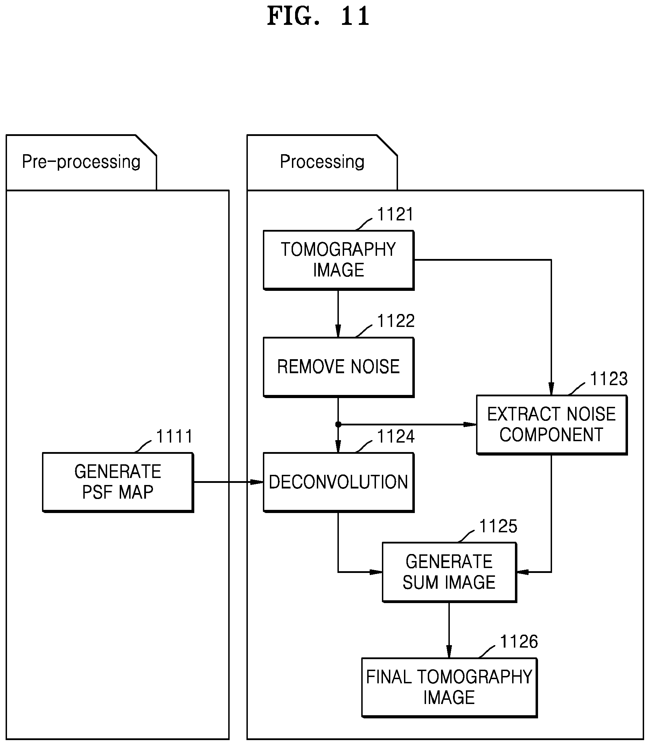

FIGS. 11 through 13 illustrate processes of acquiring a final tomography image via the tomography apparatus 1000 according to embodiments. The image processor 1020 may remove noise from an image before or after blurring the image. Alternatively, the image processor 1020 may deblur an image while removing noise therefrom by using a regularization term. The regularization term is one of noise filtering methods and is widely known to those of ordinary skill in the art, and thus a detailed description thereof will be omitted below. Embodiments are not limited to the use of a regularization term, and other general noise filtering methods may be applied.

FIG. 11 is a diagram for explaining removing of noise before deblurring an image.

Referring to FIG. 11, the controller 1010 may generate a PSF map corresponding to a position in a FOV based on a plurality of sample PSFs (1111).

The image processor 1020 may first removing noise (1122) from a tomography image 1121 and generate a difference image between the tomography image and an image from which the noise is removed (1123). In this case, the difference image may be a noise component that is present in the tomography image 1121.

The image processor 1020 may then perform deblurring on the image from which the noise is removed, based on the PSF map generated by the controller 1010 (1124). Furthermore, the image processor 1020 may generate a sum image by adding the noise component to the deblurred image in order to acquire an image that appears more natural to the user (1125). The image processor 1020 may acquire the sum image as a final tomography image (1126).

Unlike in FIG. 11, the image processor 1020 may perform removing noise after deblurring a tomography image.

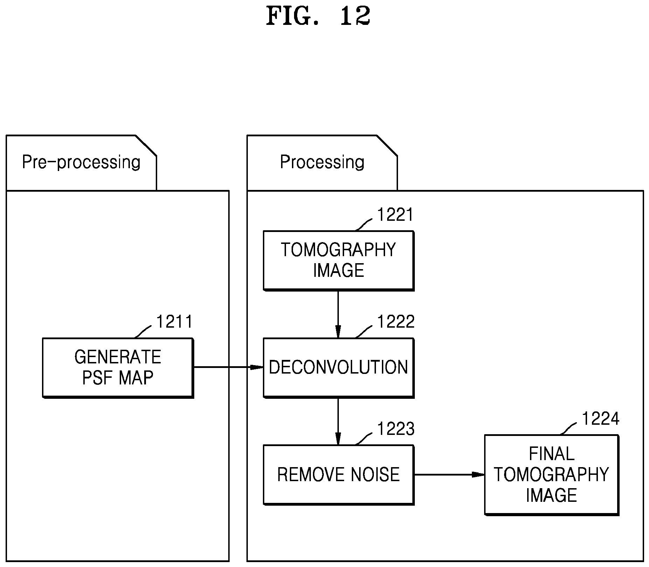

Referring to FIG. 12, the image processor 1020 may deblur a tomography image 1221 based on a PSF map (1222).

The image processor 1020 may then perform removing noise on the deblurred image (1223) to acquire a final tomography image (1224).

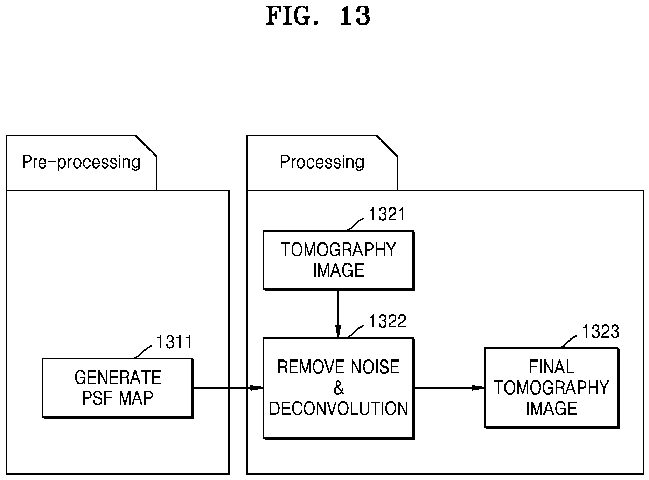

Referring to FIG. 13, unlike in FIG. 11 or 12, the image processor 1020 may perform removing noise while deblurring a tomography image 1321.

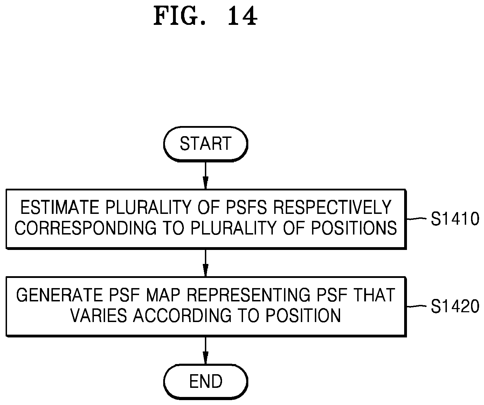

FIG. 14 is a flowchart of a method of generating a PSF map according to an embodiment.

Referring to FIG. 14, a tomography apparatus may estimate a plurality of PSFs respectively corresponding to a plurality of different positions, based on a plurality of sample images acquired by performing a tomography scan on a sample object at the different positions, respectively (S1410). In this case, the tomography apparatus may estimate each PSF as a form of Gaussian function with respect to a position, based on the plurality of sample images.

The tomography apparatus may generate, based on the estimated plurality of PSFs, a PSF map representing PSF that vary according to a position in a FOV created within a gantry (S1420).

For example, the tomography apparatus may generate a PSF map by applying interpolation or extrapolation to the plurality of PSFs, but embodiments are not limited thereto.

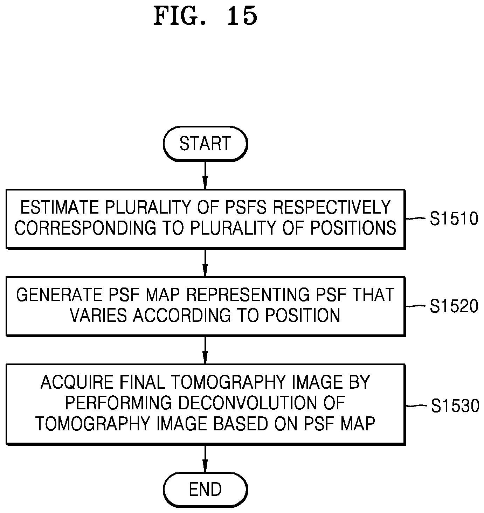

FIG. 15 is flowchart of a method of processing a tomography image according to an embodiment.

Since the method of processing a tomography image according to the present embodiment includes the same operations as those of the tomography apparatus 500 (1000) described with reference to FIGS. 1 through 13, the same descriptions as already provided with reference to FIGS. 1 through 13 will be omitted below. Referring to FIG. 15, according to the method, a plurality of PSFs respectively corresponding to a plurality of different positions are estimated based on a plurality of sample images acquired by performing a tomography scan on a sample object at the different positions, respectively (S1510). Operation S1510 may be performed by the controller 510 of the tomography apparatus 500.

Then, a PSF map representing PSF that vary according to a position is generated based on the estimated plurality of PSFs (S1520).

Thereafter, a final tomography image is acquired by deblurring a tomography image based on the generated PSF map (S1530). In detail, deconvolution of a tomography image based on the generated PSF map may reduce blurring artifacts in the tomography image. Operation S1530 may be performed by the image processor 520 of the tomography apparatus 500.

According to the method, a noise component is removed from a tomography image, and the resultant tomography image may be deblurred based on a PSF map.

Furthermore, a noise component may be acquired by generating a difference image between a tomography image and an image from which the noise component is removed. Then, a sum image generated by adding the acquired noise component to a deblurred image may be acquired as a final tomography image. In this case, the removing of the noise component may be performed before or after deblurring the image. Furthermore, according to the method, the removing of the noise component may be performed using a regularization term while deblurring the image.

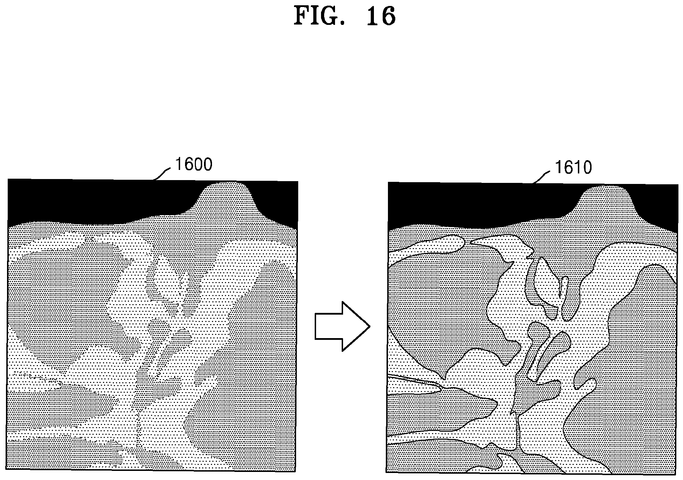

FIG. 16 is a diagram for explaining reduction of blurring artifacts in a tomography image according to a tomography apparatus and method of processing a tomography image according to embodiments.

Referring to FIG. 16, an image 1600 on the left side is an example of a tomography image reconstructed using a general method. For example, the image 1600 may be reconstructed using a FBP method. In this case, the image 1600 shows a blurry contour of an object due to the presence of blurring artifacts therein.

An image 1610 on the right side is an example of an image in which blurring artifacts are reduced by a tomography apparatus and method of processing a tomography image according to embodiments. The image 1610 depicts a clear contour of the object due to effective reduction of the blurring artifacts, and a noise component is not boosted in the image 1610.

As described above, in the tomography apparatuses and methods of processing a tomography image according to the embodiments, blurring artifacts that are present in a tomography image may be effectively reduced by generating a different PSF map according to a position in a FOV. Furthermore, boosting of noise may be prevented during deblurring of an image by processing a noise component in the image separately from a portion representing an object.

The embodiments of the inventive concept may be written as computer programs and may be implemented in general-use digital computers that execute the programs using a non-transitory computer-readable recording medium.

Examples of the non-transitory computer-readable recording medium include magnetic storage media (e.g., ROM, floppy disks, hard disks, etc.), optical recording media (e.g., CD-ROMs, or DVDs), etc.

While one or more embodiments of the inventive concept have been described with reference to the figures, it will be understood by those of ordinary skill in the art that various changes in form and details may be made therein without departing from the scope of the inventive concept as defined by the following claims. Accordingly, the above embodiments and all aspects thereof are examples only and are not limiting.

## Claims

1. A tomography apparatus (500) comprises:
a controller (510) configured to acquire a point spread function (PSF) map representing a PSF that varies according to a position in a field of view (FOV) created within a gantry; and
an image processor (520) configured to acquire a final tomography image by deblurring a tomography image based on the acquired PSF map,
**characterized in that**
the image processor (520) is further configured to remove a noise component from the tomography image and deblur the tomography image from which the noise component is removed, based on the acquired PSF map.

2. The tomography apparatus (500) of claim 1, wherein the controller (510) estimates a plurality of PSFs respectively corresponding to a plurality of different positions, based on a plurality of sample images acquired by performing a tomography scan on a sample object at the plurality of different positions, respectively, and generates the PSF map based on the estimated plurality of PSFs.

3. The tomography apparatus (500) of claim 2, wherein the controller (510) estimates, based on the plurality of sample images, each PSF as a form of a Gaussian function with respect to a position.

4. The tomography apparatus (500) of claim 2, wherein the controller (510) generates the PSF map by applying interpolation to the plurality of PSFs.

5. The tomography apparatus (500) of claim 2, wherein the controller (510) generates the PSF map by applying extrapolation to the plurality of PSFs.

6. The tomography apparatus (500) of claim 1, further comprising a data acquisition device configured to acquire tomography data by performing a tomography scan on an object,
wherein the image processor (520) reconstructs the tomography image based on the acquired tomography data and acquires the final tomography image by deblurring the tomography image, based on the PSF map.

7. The tomography apparatus (500) of claim 1, wherein the image processor (520) acquires the noise component by generating a difference image between the tomography image and the tomography image from which the noise component is removed and acquires as the final tomography image a sum image generated by adding the acquired noise component to the deblurred tomography image.

8. The tomography apparatus (500) of claim 1, further comprising a storage device configured to store the PSF.

9. A method of processing a tomography image, the method comprises:
acquiring a point spread function (PSF) map representing a PSF that varies according to a position in a FOV created within a gantry; and
acquiring a final tomography image by deblurring the tomography image, based on the acquired PSF map
**characterized in that**
the acquiring of the final tomography image comprises:
removing a noise component from the tomography image; and
deblurring the tomography image from which the noise component is removed, based on the acquired PSF map.

10. The method of claim 9, wherein the acquiring of the PSF map comprises:
estimating a plurality of PSFs respectively corresponding to a plurality of different positions, based on a plurality of sample images acquired by performing a tomography scan on a sample object at the plurality of different positions, respectively; and
generating the PSF map based on the estimated plurality of PSFs.

11. The method of claim 10, wherein the estimating of the plurality of PSFs comprises estimating, based on the plurality of sample images, each PSF as a form of a Gaussian function with respect to a position.

12. The method of claim 9, wherein the acquiring of the final tomography image comprises:
reconstructing the tomography image based on tomography data acquired by performing a tomography scan on an object; and
acquiring the final tomography image by deblurring the tomography image based on the PSF map.

13. The method of claim 9, wherein the acquiring of the final tomography image comprises:
acquiring a noise component by generating a difference image between the tomography image and the tomography image from which the noise component is removed; and
acquiring as the final tomography image a sum image produced by adding the acquired noise component to the deblurred tomography image.

## Patentansprüche

1. Tomographieeinrichtung (500), umfassend:
eine Steuerung (510), die dazu ausgestaltet ist, eine Punktspreizfunktions (PSF-point spread function) Abbildung zu erfassen, die eine PSF darstellt, die gemäß einer Position in einem Sichtfeld (FOV-field of view) variiert, das innerhalb einer Gantry erzeugt wird; und
einen Bildprozessor (520), der dazu ausgestaltet ist, durch Schärfen eines Tomographiebilds auf Basis der erfassten PSF-Abbildung ein finales Tomographiebild zu erfassen,
**dadurch gekennzeichnet, dass**
der Bildprozessor (520) ferner dazu ausgestaltet ist, eine Rauschkomponente aus dem Tomographiebild zu entfernen und das Tomographiebild, aus dem die Rauschkomponente entfernt worden ist, auf Basis der erfassten PSF-Abbildung zu schärfen.

2. Tomographieeinrichtung (500) nach Anspruch 1, wobei die Steuerung (510) eine Vielzahl von PSFs, die jeweils einer Vielzahl von verschiedenen Positionen entsprechen, auf Basis einer Vielzahl von Musterbildern schätzt, die jeweils durch das Durchführen eines Tomographiescans an einem Musterobjekt an der Vielzahl von verschiedenen Positionen erfasst werden, und auf Basis der geschätzten Vielzahl von PSFs die PSF-Abbildung generiert.

3. Tomographieeinrichtung (500) nach Anspruch 2, wobei die Steuerung (510) auf Basis der Vielzahl von Musterbildern jede PSF in Form einer Gaußfunktion in Bezug auf eine Position schätzt.

4. Tomographieeinrichtung (500) nach Anspruch 2, wobei die Steuerung (510) die PSF-Abbildung unter Anwendung von Interpolation auf die Vielzahl von PSFs generiert.

5. Tomographieeinrichtung (500) nach Anspruch 2, wobei die Steuerung (510) die PSF-Abbildung unter Anwendung von Extrapolation auf die Vielzahl von PSFs generiert.

6. Tomographieeinrichtung (500) nach Anspruch 1, ferner umfassend eine Datenerfassungsvorrichtung, die dazu ausgestaltet ist, durch das Durchführen eines Tomographiescans an einem Objekt Tomographiedaten zu erfassen,
wobei der Bildprozessor (520) das Tomographiebild auf Basis der erfassten Tomographiedaten rekonstruiert und das finale Tomographiebild durch Schärfen des Tomographiebilds auf Basis der PSF-Abbildung erfasst.

7. Tomographieeinrichtung (500) nach Anspruch 1, wobei der Bildprozessor (520) die Rauschkomponente durch Generieren eines Differenzbilds zwischen dem Tomographiebild und dem Tomographiebild erfasst, aus dem die Rauschkomponente entfernt worden ist, und als das finale Tomographiebild ein Summenbild erfasst, das durch Addieren der erfassten Rauschkomponente zu dem geschärften Tomographiebild generiert wird.

8. Tomographieeinrichtung (500) nach Anspruch 1, ferner umfassend eine Speichervorrichtung, die dazu ausgestaltet ist, die PSF zu speichern.

9. Verfahren zur Verarbeitung eines Tomographiebilds, wobei das Verfahren Folgendes umfasst:
Erfassen einer Punktspreizfunktions (PSF)Abbildung, die eine PSF darstellt, die gemäß einer Position in einem Sichtfeld variiert, das innerhalb einer Gantry erzeugt wird; und
Erfassen eines finalen Tomographiebilds durch Schärfen des Tomographiebilds auf Basis der erfassten PSF-Abbildung
**dadurch gekennzeichnet, dass**
das Erfassen des finalen Tomographiebilds Folgendes umfasst:
Entfernen einer Rauschkomponente aus dem Tomographiebild; und
Schärfen des Tomographiebilds, aus dem die Rauschkomponente entfernt worden ist, auf Basis der erfassten PSF-Abbildung.

10. Verfahren nach Anspruch 9, wobei das Erfassen der PSF-Abbildung Folgendes umfasst:
Schätzen einer Vielzahl von PSFs, die jeweils einer Vielzahl von verschiedenen Positionen entsprechen, auf Basis einer Vielzahl von Musterbildern, die jeweils durch das Durchführen eines Tomographiescans an einem Musterobjekt an der Vielzahl von verschiedenen Positionen erfasst werden; und
Generieren der PSF-Abbildung auf Basis der geschätzten Vielzahl von PSFs.

11. Verfahren nach Anspruch 10, wobei das Schätzen der Vielzahl von PSFs das Schätzen jeder PSF in Form einer Gaußfunktion in Bezug auf eine Position auf Basis der Vielzahl von Musterbildern umfasst.

12. Verfahren nach Anspruch 9, wobei das Erfassen des finalen Tomographiebilds Folgendes umfasst:
Rekonstruieren des Tomographiebilds auf Basis von Tomographiedaten, die durch das Durchführen eines Tomographiescans an einem Objekt erfasst werden; und
Erfassen des finalen Tomographiebilds durch Schärfen des Tomographiebilds auf Basis der PSF-Abbildung.

13. Verfahren nach Anspruch 9, wobei das Erfassen des finalen Tomographiebilds Folgendes umfasst:
Erfassen einer Rauschkomponente durch Generieren eines Differenzbilds zwischen dem Tomographiebild und dem Tomographiebild, aus dem die Rauschkomponente entfernt worden ist; und
Erfassen, als das finale Tomographiebild, eines Summenbilds, das durch Addieren der erfassten Rauschkomponente zu dem geschärften Tomographiebild erstellt wird.

## Revendications

1. Appareil de tomographie (500) comprenant :
un contrôleur (510) conçu pour acquérir une carte de fonction d'étalement du point (PSF-point spread function) représentant une PSF qui varie en fonction d'une position dans un champ de vision (FOV-field of view) créé dans un portique ; et
un processeur d'images (520) conçu pour acquérir une image tomographique finale par le défloutage d'une image tomographique basé sur la carte PSF acquise,
**caractérisé en ce que**
le processeur d'images (520) est conçu en outre pour éliminer une composante de bruit de l'image tomographique et pour déflouter l'image tomographique de laquelle la composante de bruit est éliminée, basé sur la carte PSF acquise.

2. Appareil de tomographie (500) selon la revendication 1, le contrôleur (510) estimant une pluralité de PSF correspondant respectivement à une pluralité de positions différentes, basé sur une pluralité d'images échantillons acquises en réalisant un examen tomographique sur un objet échantillon en une pluralité de positions différentes, respectivement, et générant la carte PSF basé sur la pluralité estimée des PSF.

3. Appareil de tomographie (500) selon la revendication 2, le contrôleur (510) estimant, chaque PSF comme une forme d'une fonction gaussienne relativement à une position, basé sur la pluralité d'images échantillons.

4. Appareil de tomographie (500) selon la revendication 2, le contrôleur (510) générant la carte PSF en appliquant une interpolation à la pluralité des PSF.

5. Appareil de tomographie (500) selon la revendication 2, le contrôleur (510) générant la carte PSF en appliquant une extrapolation à la pluralité des PSF.

6. Appareil de tomographie (500) selon la revendication 1, comprenant en outre un dispositif d'acquisition de données conçu pour acquérir des données de tomographie en réalisant un examen tomographique sur un objet,
le processeur d'images (520) reconstruisant l'image tomographique basé sur les données de tomographie acquises et acquérant l'image tomographique finale par le défloutage de l'image tomographique, basé sur la carte PSF.

7. Appareil de tomographie (500) selon la revendication 1, le processeur d'images (520) acquérant la composante de bruit en générant une image de différence entre l'image tomographique et l'image tomographique de laquelle la composante de bruit est éliminée et acquérant comme image tomographique une image somme générée en ajoutant à l'image tomographique défloutée la composante de bruit acquise.

8. Appareil de tomographie (500) selon la revendication 1, comprenant en outre un dispositif d'enregistrement conçu pour enregistrer la PSF.

9. Procédé de traitement d'une image tomographique, le procédé comprenant :
acquérir une carte de fonction d'étalement du point (PSF) représentant une PSF qui varie en fonction d'une position dans un FOV créé dans un portique ; et
acquérir une image tomographique finale par le défloutage d'une image tomographique, basé sur la carte PSF acquise,
**caractérisé en ce que**
l'acquisition de l'image tomographique finale comprend :
éliminer une composante de bruit de l'image tomographique ; et
déflouter une image tomographique de laquelle la composante de bruit est éliminée, basé sur la carte PSF acquise.

10. Procédé selon la revendication 9, l'acquisition de la carte PSF comprenant : estimer une pluralité de PSF correspondant respectivement à une pluralité de positions différentes, basé sur une pluralité d'images échantillons acquises en réalisant un examen tomographique sur un objet échantillon en une pluralité de positions différentes, respectivement ; et générer la carte PSF basé sur la pluralité estimée des PSF.

11. Procédé selon la revendication 10, l'estimation de la pluralité de PSF comprenant l'estimation, basé sur la pluralité d'images échantillons, de chaque PSF comme une forme d'une fonction gaussienne relativement à une position.

12. Procédé selon la revendication 9, l'acquisition de l'image tomographique finale comprenant :
reconstruire l'image tomographique basé sur des données de tomographie acquises en réalisant un examen tomographique sur un objet ; et
acquérir l'image tomographique finale par le défloutage de l'image tomographique, basé sur la carte PSF.

13. Procédé selon la revendication 9, l'acquisition de l'image tomographique finale comprenant :
acquérir une composante de bruit en générant une image de différence entre l'image tomographique et l'image tomographique de laquelle la composante de bruit est éliminée ; et
acquérir en tant qu'image tomographique finale une image somme produite en ajoutant à l'image tomographique défloutée la composante de bruit acquise.
